# EUROPEAN PATENT SPECIFICATION

(11) **EP 1 788 075 B1**
(45) Date of publication and mention of the grant of the patent: **17.02.2010**
(21) Application number: 05776635.4
(22) Date of filing: 01.09.2005
(51) Int. Cl.: C12N 1/21, C07G 13/00, C12N 15/09, C12P 7/40, C12P 7/64, C12P 13/00, C12P 13/04, C12P 19/00, C12P 19/28

(54) **INDUSTRIALLY USEFUL MICROORGANISM**
GROSSTECHNISCH GEEIGNETER MIKROORGANISMUS
MICRO-ORGANISME D'UTILITE INDUSTRIELLE

(30) Priority: 01.09.2004 JP 2004254446
(43) Date of publication of application: 23.05.2007
(73) Proprietor: Kyowa Hakko Kirin Co., Ltd., Tokyo (JP)
(72) Inventor: KATO, Makiko, Tokyo 194-0022 (JP); MORI, Hideo, Tokyo 194-0023 (JP); KATO, Jun-ichi, Yokohama-shi, Kanagawa 222-0022 (JP)
(74) Representative: Harding, Charles Thomas
(86) International application number: PCT/JP2005/015977
(87) International publication number: WO 2006/025477

(56) References cited:
- MORENO ALBERTO J ET AL: "ihfA gene of the bacterium Myxococcus xanthus and its role in activation of carotenoid genes by blue light" JOURNAL OF BACTERIOLOGY, vol. 183, no. 2, January 2001 (2001-01), pages 557-569, XP002444047 ISSN: 0021-9193
- MILLER H I: "MULTI LEVEL REGULATION OF BACTERIO PHAGE LAMBDA LYSOGENY BY THE ESCHERICHIA-COLI HIMA GENE" CELL, vol. 25, no. 1, 1981, pages 269-276, XP002444048 ISSN: 0092-8674
- BYKOWSKI TOMASZ ET AL: "Selected phenotypes of ihf mutants of Escherichia coli" BIOCHIMIE (PARIS), vol. 80, no. 12, December 1998 (1998-12), pages 987-1001, XP002444567 ISSN: 0300-9084
- PALACIOS SERGIO ET AL: "prpR, ntrA, and ihf functions are required for expression of the prpBCDE operon, encoding enzymes that catabolize propionate in Salmonella enterica serovar Typhimurium LT2" JOURNAL OF BACTERIOLOGY, vol. 182, no. 4, February 2000 (2000-02), pages 905-910, XP002444568 ISSN: 0021-9193
- COMPAN I. ET AL: 'Interaction of six global transcription regulators in expression of manganese superoxide dismutase in Escherichia coli K-12' J. BACTERIOL. vol. 175, no. 6, 1993, pages 1687 - 1696, XP002994828
- PRESUTTI D.G. ET AL: 'Binding of integration host factor (IHF) to the Escherichia coli sodA gene and its role in the regulation of a sodA-lacZ fusion gene' MOL. GEN. GENET. vol. 246, no. 2, 1995, pages 228 - 235, XP002994829
- HUANG L. ET AL: 'Integration host factor is a negative effector of in vivo and in vitro expression of ompC in Escherichia coli' J. BACTERIOL. vol. 172, no. 9, 1990, pages 5293 - 5298, XP002994830

## Description

### Technical Field

The present invention relates to a process for producing a useful substance using a microorganism capable of producing a useful substance.

### Background Art

Up to the present, numerous reports have been made concerning processes for producing useful substances, such as amino acids, with the use of a microorganism. Most of such techniques involve the use of (1) a microorganism in which the expression level of a biosynthetic gene for a useful substance is increased, (2) a microorganism having a desensitized mutant gene of said gene, or (3) a microorganism in which a gene of an enzyme degrading a useful substance is disrupted (Non-Patent Documents 1 to 3).

As an example of a process for producing useful substances using microorganisms other than (1) to (3) above, a process for producing lysine using *Escherichia coli* in which the *rmf* gene that encode proteins having activity of lowering growth rate is disrupted is known (Patent Document 1). In general, however, it is difficult to identify genes that are indirectly involved with the acceleration of biosynthesis or the inhibition of degradation of useful substances.

The entire nucleotide sequences of the chromosomal DNAs of many microorganisms have been elucidated (Non-Patent Document 4). Also, a method wherein a given gene or a given region on chromosomal DNA of a microorganism is deleted as designed by homologous recombination has been known (Non-Patent Document 5). With the utilization of the entire nucleotide sequence information of chromosomal DNA and homologous recombination, a library of mutant microorganisms in which genes of chromosomal DNA are comprehensively disrupted, a library of mutant microorganisms in which approximately 20-kbp deletable regions of chromosomal DNAs are comprehensively deleted, and the like have been constructed (Non-Patent Documents 6 and 7).

The amino acid sequences of proteins constituting the *E. coli* integration host factors (complexes of the *ihfA* (*himA*) gene products and the *ihfB* (*himD*) gene products; hereafter referred to as the *ihfAB* gene product complexes) and the nucleotide sequences of the genes encoding the proteins are already known (Non-Patent Document 8). It is also known that the *ihfAB* gene product complexes bind to various sites on the *E. coli* chromosome and regulate the transcription of various genes of *E. coli* (Non-Patent Document 9), although the correlation between activity of the *ihfAB* gene product complexes and the capacity for producing a useful substance is not yet known.
Patent-Document 1: JP Patent Publication (kokai) No. 2002-306191 A
Non-Patent Document 1: Advances in Biochemical Engineering/Biotechnology, 79, Springer, 2003
Non-Patent Document 2: Kakusan hakkou (Nucleic acid fermentation), Kodansha Scientific Ltd., 1976
Non-Patent Document 3: J. Biosci. Bioeng., 90, 522, 2000
Non-Patent Document 4: http://www.tigr.org/tdb/mdb/mdbcomplete.html
Non-Patent Document 5: J. Bacteriol., 180, 2063, 1998
Non-Patent Document 6: Tanpakushitsu, kakusan, kouso (Protein, nucleic acid, enzyme), vol. 46, 2386, 2001
Non-Patent Document 7: Nature Biotechnol., 20, 1018, 2002
Non-Patent Document 8: Science, 277, 1453, 1997
Non-Patent Document 9: J. Bacteriol., 181, 3246,1999

### Disclosure of the Invention

### Object to be Solved by the Invention

It is an object of the present invention to provide a method for producing a useful substance using a microorganism with the increased capacity for producing a useful substance.

### Means for Attaining the Object

The present invention relates to the following (6).
(1) A microorganism which comprises chromosomal DNA lacking a part or entire of the gene encoding a protein having the amino acid sequence as shown in at least one of SEQ ID NOs: 1 and 2 or the gene encoding a protein having 80% or more homology with the amino acid sequence as shown in at least one of SEQ ID NOs: 1 and 2, and which has the ability to produce the amino acid.
(2) The microorganism according to (1) and (4), wherein the gene encoding a protein having the amino acid sequence as shown in SEQ ID NO: 1 or 2 has the nucleotide sequence as shown in SEQ ID NO: 3 or 4, respectively.
(3) The microorganism according to (1) and (4) or (2), wherein the ability to produce a useful substance is enhanced by a method selected from among the following [1] to [5]:
   [1] a method for reducing or removing at least one mechanism for regulating biosynthesis of the useful substance;
   [2] a method for increasing the expression level of at least one enzyme associated with biosynthesis of the useful substance;
   [3] a method for increasing the copy number of at least one enzyme gene associated with biosynthesis of the useful substance;
   [4] a method for attenuating or blocking at least one metabolic pathway which branches from the biosynthetic pathway of the useful substance into metabolites other than the useful substance; or
   [5] a method for selecting a strain that exhibits higher resistance to an analogue of a useful substance than a wild-type strain.
(4) The microorganism according to any one of (1) to (3), which belongs to the genus *Erwinia, Serratia, Salmonella, Escherichia, Proteus, Pseudomonas*, or *Xanthomonas.*
(5) The microorganism according to any one of (1) to (4), which is selected from the group consisting of *Erwinia berbicola, Erwinia amylovora, Erwinia carotovora, Serratia marcescens, Serratia ficaria, Serratia fonticola, Serratia liquefaciens, Salmonella typhimurium, Escherichia coli, Proteus rettgeri, Pseudomonas putida, Pseudomonas fluorescens, Pseudomonas aeruginosa, Pseudomonas dacunhae, Pseudomonas thazdinophilum, Xanthomonas oryzae*, and *Xanthomonas capestris.*
(6) A process for producing an amino acid which comprises culturing a microorganism in a medium so as to form and accumulate the amino acid in a culture product and recovering the amino acid from the culture product, wherein the microorganism is a microorganism according to (1) and (4), preferably (2), (3) or (5). Also described is (7) the process according to any one of (1) to (6), wherein the useful substance is selected from the group consisting of proteins, peptides, amino acids, nucleic acids, vitamins, saccharides, organic acids, and lipids.

### Effects of the Invention

The present invention can provide a method for producing a useful substance using a microorganism with enhanced ability to produce a useful substance.

### Preferred Embodiments of the Invention

### 1. Microorganisms described herein

The microorganism described herein has chromosomal DNA that lacks a part or entire of the gene encoding a protein (*IhfA* or *IhfB* protein) having the amino acid sequence as shown in SEQ ID NO: 1 or 2 or the gene encoding a protein having 80% or more, preferably 90% or more, more preferably 95% or more, further preferably 98% or more, and particularly preferably 99% or more homology with the amino acid sequence as shown in SEQ ID NO: 1 or 2, and are capable of producing a useful substance. In this description, a gene comprises a structural gene and a region that has a given regulatory function, such as a promoter or operator region.

Homology of amino acid sequence or nucleotide sequence can be determined with the utilization of the BLAST algorithm of Karlin and Altschul [Pro. Natl. Acad. Sci., U.S.A., 90, 5873, 1993] or the FASTA algorithm [Methods Enzymol., 183, 63, 1990]. Based on the BLAST algorithm, programs referred to as BLASTN or BLASTX have been developed [J. Mol. Biol., 215, 403, 1990]. When analyzing nucleotide sequences by BLASTN based on BLAST, the score parameter is set to 100 and the word length parameter is set to 12, for example. When analyzing amino acid sequences by BLASTX based on BLAST, the score parameter is set to 50 and the word length parameter is set to 3, for example. When using BLAST and Gapped BLAST programs, the default parameters thereof are used. Specific procedures of such analytical methods are known (http://www.ncbi.nlm.nih.gov.).

A microorganism which comprises chromosomal DNA that lacks a part or entire of the gene encoding a protein having the amino acid sequence as shown in SEQ ID NO: 1 or 2 or the gene encoding a protein having 80% or more homology with the amino acid sequence as shown in SEQ ID NO: 1 or 2 (hereafter referred to as the *ihf* gene or a homologous gene thereof) involves deletion, substitution or addition of nucleotide(s) in the nucleotide sequence of the gene on chromosomal DNA. Examples of such microorganism include (1) a microorganism in which transcription regulatory functions of promoters, operators or the like of the *ihf* gene or a homologous gene thereof do not function and a protein encoded by the *ihf* gene or a homologous gene thereof (hereafter referred to as the Ihf protein or a homologous protein thereof) is not expressed; (2) a microorganism in which frame shift takes place and the Ihf protein or a homologous protein thereof is not expressed as an active protein; and (3) a microorganism that lacks a part or entire of the sequence of the structural gene of the *ihf* gene or a homologous gene thereof, resulting in a lack of expression of the Ihf protein or a homologous protein thereof as an active protein; with microorganisms of the item (3) being preferable.

A specific example of the microorganisms of the item (3) is a microorganism in which the Ihf protein or a homologous protein thereof is not expressed as an active protein due to a lack of a part or entire of the sequence of the structural gene in the nucleotide sequence as shown in SEQ ID NO: 3 or 4. The term "lack of a part of the sequence" may refer to a lack of a single nucleotide in the structural gene portion of the nucleotide sequence as shown in SEQ ID NO: 3 or 4, provided that a protein having the amino acid sequence as shown in SEQ ID NO: 1 or 2 loses its activity due to such lack. The structural gene of the gene comprising the nucleotide sequence as shown in SEQ ID NO: 3 or 4 can preferably lack 5 to 10 nucleotides, more preferably 10 to 50 nucleotides, and further preferably 50 to 100 nucleotides.

Useful substances produced by the microorganisms may be any substances, as long as they can be produced by microorganisms and they are considered to be industrially useful. Examples of preferable substances include proteins, peptides, amino acids, nucleic acids, vitamins, saccharides, organic acids, and lipids. Preferred examples of proteins include inosine kinase, glutamate 5-kinase (EC 2.7.2.11), glutamate-5-semialdehyde dehydrogenase (EC 1.2.1.41), pyrroline-5-carboxylate reductase (EC 1.5.1.2), and human granulocyte colony-stimulating factors. Preferred example of peptides is glutathione. Preferred examples of amino acids include L-alanine, glycine, L-glutamine, L-glutamic acid, L-asparagine, L-aspartic acid, L-lysine, L-methionine, L-threonine, L-leucine, L-valine, L-isoleucine, L-proline, L-histidine, L-arginine, L-tyrosine, L-tryptophan, L-phenylalanine, L-serine, L-cysteine, L-3-hydroxyproline, and L-4-hydroxyproline. Preferred examples of nucleic acids include inosine, guanosine, inosinic acid, and guanylic acid. Preferred examples of vitamins include riboflavin, thiamine, and ascorbic acid. Preferred examples of saccharides include xylose and xylitol. Preferred examples of lipids include eicosapentaenoic acid (EPA) and docosahexaenoic acid (DHA).

The microorganism may belong to any genus, provided that such microorganism lacks a part or entire of the sequence of the *ihf* gene of the chromosomal DNA or a homologous gene thereof and is capable of producing a useful substance, as described above. The microorganism is preferably a prokaryote, and more preferably a bacterium.

For example, the microorganism can preferably belong to the bacterial genus *Erwinia, Serratia, Salmonella, Escherichia, Proteus, Pseudomonas*, or *Xanthomonas.* More preferably, the microorganism can belong to the species *Erwinia berbicola, Erwinia amylovora, Erwinia carotovora, Serratia marcescens, Serratia ficaria, Serratia fonticola, Serratia liquefaciens, Salmonella typhimurium, Escherichia coli, Proteus rettgeri, Pseudomonas putida, Pseudomonas fluorescens, Pseudomonas aeruginosa, Pseudomonas dacunhae, Pseudomonas thazdinophilum, Xanthomonas oryzae*, or *Xanthomonas capestris,* among which *E. coli* is particularly preferable.

### 2. A method for preparing the microorganism

The microorganism can be produced by (1) a method wherein a part or entire of the sequence of the *ihf* gene which is present on chromosomal DNA of a microorganism capable of producing a useful substance, or a homologous gene thereof is deleted, or (2) a method wherein the ability to produce a useful substance is imparted to a microorganism that lacks a part or entire of the sequence of the *ihf* gene which is present on chromosomal DNA, or a homologous gene thereof.

A microorganism capable of producing a useful substance may be any microorganism, provided that it is capable of producing one or more types of useful substances. Examples of such microorganism include a microorganism isolated from nature that is capable of producing a useful substance, and a microorganism to which the ability to produce a desirable useful substance has been artificially imparted by conventional methods.

Examples of such conventional methods include:
(a) a method for reducing or removing at least one mechanism for regulating biosynthesis of a useful substance;
(b) a method for increasing the expression level of at least one enzyme associated with biosynthesis of a useful substance;
(c) a method for increasing the copy number of at least one enzyme gene associated with biosynthesis of a useful substance;
(d) a method for attenuating or blocking at least one metabolic pathway which branches from a biosynthetic pathway of a useful substance into metabolites other than the useful substance; and
(e) a method for selecting a strain that exhibits higher resistance to an analogue of a useful substance than a wild-type strain.
Such conventional methods can be carried out alone or in combinations of two or more.

When useful substances are amino acids, for example, method (a) is described in Agric. Biol. Chem., 43, 105-111, 1979, J. Bacteriol., 110, 761-763, 1972, and Appl. Microbiol. Biotechnol., 39, 318-323, 1993; method (b) is described in Agric. Biol. Chem., 43, 105-111, 1979 and J. Bacteriol., 110, 761-763, 1972; method (c) is described in Appl. Microbiol. Biotechnol., 39, 318-323,1993 and Agric. Biol. Chem., 39, 371-377, 1987; method (d) is described in Appl. Environ. Microbiol., 38, 181-190, 1979 and Agric. Biol. Chem., 42, 1773-1778, 1978; and method (e) is described in Agric. Biol. Chem., 36, 1675-1684, 1972, Agric. Biol. Chem., 41, 109-116, 1977, Agric. Biol. Chem., 37, 2013-2023, 1973, and Agric. Biol. Chem., 51, 2089-2094, 1987. With reference to these literatures, microorganisms capable of forming and accumulating various amino acids can be produced.

Also, many examples of a method for producing microorganisms capable of forming and accumulating amino acids by any of or two or more of methods (a) to (e) above are described in Biotechnology 2nd ed., vol. 6, Products of Primary Metabolism (VCH Verlagsgesellschaft mbH, Weinheim, 1996), section 14a or 14b, Advances in Biochemical Engineering/Biotechnology 79, 1-35, 2003, or Amino san Hakko (Amino acid fermentation), Japan Scientific Societies Press, Hiroshi Aida et al. (1986). In addition, many reports have been made concerning a specific method for producing microorganisms capable of forming and accumulating amino acids. Examples thereof include Japanese Published and Unexamined Patent Application No. 2003-164297, Agric. Biol. Chem., 39, 153-160, 1975, Agric. Biol. Chem., 39, 1149-1153, 1975, Japanese Published and Unexamined Patent Application No. 13599/83(1983), J. Gen. Appl. Microbiol., 4, 272-283, 1958, Japanese Published and Unexamined Patent Application No. 94985/88(1988), Agric. Biol. Chem., 37, 2013-2023, 1973, WO 97/15673, Japanese Published and Unexamined Patent Application No. 18596/81(1981), Japanese Published and Unexamined Patent Application No. 144092/81(1981), and Japanese Published and Unexamined Patent Application No. 2003-511086. With reference to these literatures, microorganisms capable of producing one or more types of amino acids can be produced.

Microorganisms that lack a part or entire of the sequence of the *ihf* gene on chromosomal DNA or a homologous gene thereof can be obtained by any method without limitation, so long as such microorganism can be obtained. For example, with the utilization of nucleotide sequence information of a gene encoding a protein having the amino acid sequence as shown in SEQ ID NO: 1 or 2 on chromosomal DNA of the microorganism given below or a homologous gene of the *ihf* gene*,* microorganisms of interest can be obtained by a method of introducing deletion, substitution or addition of nucleotide(s) into the *ihf* gene of chromosomal DNA of the microorganism or a homologous gene thereof. The nucleotide sequence of the homologous gene of the *ihf* gene can be determined in the following manner. That is, the homologous gene of the *ihf* gene is identified and obtained by Southern hybridization of chromosomal DNAs of various microorganisms using probes comprising all or part of the DNA having the nucleotide sequence as shown in SEQ ID NO: 3 or 4, or the homologous gene of the *ihf* gene is identified and obtained by PCR using primer DNA designed based on the nucleotide sequence as shown in SEQ ID NO: 3 or 4 and as templates chromosomal DNAs of various microorganisms. Thereafter, the nucleotide sequence of such gene is analyzed by a conventional method. Chromosomal DNAs that are subjected to Southern hybridization or PCR can be obtained from any microorganisms. Preferably, such microorganisms belong to the genus *Erwinia, Serratia, Salmonella, Escherichia, Proteus, Pseudomonas*, or *Xanthomonas.* More preferably, chromosomal DNAs are obtained from microorganisms that belong to the species *Erwinia berbicola, Erwinia amylovora, Erwinia carotovora, Serratia marcescens, Serratia ficaria, Serratia fonticola, Serratia liquefaciens, Salmonella typhimurium, Escherichia coli, Proteus rettgeri, Pseudomonas putida, Pseudomonas fluorescens, Pseudomonas aeruginosa, Pseudomonas dacunhae, Pseudomonas thazdinophilum, Xanthomonas oryzae*, or *Xanthomonas capestris.*

Specific examples of nucleotide sequences of homologous genes of the *ihf* genes include the nucleotide sequences of *ihfA* and *ihfB* genes derived from *Erwinia carotovora* (GenBank Accession No. BX950851), the nucleotide sequences of *ihfA* and *ihfB* genes derived from *Pseudomonas aeruginosa* (GenBank Accession No. AE004091), and the nucleotide sequences of *ihfA* and *ihfB* genes derived from *Xanthomonas capestris* (GenBank Accession No. AE008922).

Deletion, substitution or addition of the nucleotide(s) can be introduced into a gene of chromosomal DNA of a microorganism by a method utilizing homologous recombination, for example. An example of a method utilizing general homologous recombination is a method involving the use of plasmids for homologous recombination that can be prepared by ligating mutant gene comprising nucleotides deletion, substitution or addition to plasmid DNAs having a drug-resistant gene that cannot be autonomously replicated in a host cell into which introduction of nucleotide deletion or the like is intended.

After the plasmids for homologous recombination are introduced into a host cell by a conventional method, transformants into which the plasmids for homologous recombination have been integrated to chromosomal DNA by homologous recombination are selected using the drug resistance as an indicator. The obtained transformants are cultured in a medium that does not contain the aforementioned drug for several hours to 1 day, the cultures are applied to an agar medium that contains the drug and to an agar medium that does not contain the drug, and strains that do not grow in the former medium but grow in the latter medium are selected. Thus, strains in which the second homologous recombination has taken place on the chromosomal DNA can be obtained. By determining the nucleotide sequence of a region of the chromosomal DNA in which the gene comprising nucleotide deletion or the like is present, the introduction of deletion, substitution or addition of nucleotide(s) into the target gene on chromosomal DNA can be confirmed.

An example of a microorganism into which deletion, substitution or addition of nucleotide(s) can be introduced into the target gene on a chromosomal DNA by the above method is a microorganism that belongs to the genus *Escherichia.*

An example of a method utilizing homologous recombination, and thereby effectively introducing deletion, substitution or addition of nucleotide(s) into a plurality of genes, is a method involving the use of a linear DNA.

Specifically, a linear DNA that contains a gene into which introduction of deletion, substitution or addition of nucleotide(s) is intended is incorporated into a cell to cause homologous recombination between the chromosomal DNA and the introduced linear DNA. This method can be applied to any microorganisms, provided that such microorganisms effectively incorporate a linear DNA. Such microorganisms are preferably of the genus *Escherichia*, more preferably *Escherichia coli*, and further preferably *Escherichia coli* that expresses λ phage-derived recombinant proteins (Red recombination system), for example.

An example of *Escherichia coli* that expresses the λRed recombination system is *Escherichia coli* JM101 comprising pKD46, which is plasmid DNA having the gene of the λ Red recombination system (available from the *E. coli* Genetic Stock Center, Yale University, U.S.A.).

Examples of DNAs that can be used for homologous recombination include:
(a) a linear DNA comprising, at both ends of the drug-resistant gene, DNA existing at both outside regions of a chromosomal DNA into which the introduction of deletion, substitution or addition of nucleotide(s) is intended, or DNA homologous thereto;
(b) a linear DNA to which DNA existing at both outside regions of a chromosomal DNA into which the introduction of deletion, substitution or addition of nucleotide(s) is intended, or DNA homologous thereto is directly ligated;
(c) a linear DNA comprising, at both ends of DNA to which a drug-resistant gene and a gene that can be used for negative selection are ligated, DNA existing at both outside regions of chromosomal DNA into which the introduction of deletion, substitution or addition of nucleotide(s) is intended, or DNA homologous thereto; and
(d) a linear DNA according to (a), which further comprises a nucleotide sequence which is recognized by yeast-derived Flp recombinase [Proc. Natl. Acad. Sci., U.S.A., 82, 5875, 1985] between a drug-resistant gene and DNA existing at both outside regions of a chromosomal DNA.

Any drug-resistant genes can be used, provided that such genes impart drug resistance to a drug to which a host microorganism shows sensitivity. When *Escherichia coli* is used as a host microorganism, for example, kanamycin-resistant genes, chloramphenicol-resistant genes, gentamicin-resistant genes, spectinomycin-resistant genes, tetracycline-resistant genes, or ampicillin-resistant genes can be used as drug-resistant genes.

The genes that can be used for negative selection are genes that are lethal to microorganisms under given culture conditions, when such genes are expressed in the host microorganisms. Examples of such genes include *sacB* genes derived from the microorganism which belongs to the genus *Bacillus* [Appl. Environ. Microbiol., 59, 1361-1366, 1993] and *rpsL* genes derived from the microorganism which belongs to the genus *Escherichia* [Genomics, 72, 99-104, 2001].

DNA that exists at both ends of the aforementioned linear DNA, which is homologous to DNA located at both outside regions of a chromosomal DNA into which introduction of substitution or deletion of nucleotide(s) is intended, is oriented on the linear DNA in the same direction as the direction thereof on the chromosomal DNA. The length thereof is preferably about 10 bp to 100 bp, more preferably about 20 bp to 50 bp, and further preferably about 30 bp to 40 bp.

The nucleotide sequence that is recognized by yeast-derived Flp recombinase is not particularly limited, provided that the aforementioned protein recognizes such nucleotide sequence and catalyzes homologous recombination of such nucleotide sequence. Preferable examples include DNA having the nucleotide sequence as shown in SEQ ID NO: 7 and DNA having a nucleotide sequence derived from the nucleotide sequence as shown in SEQ ID NO: 7 by deletion, substitution, or addition of one to several nucleotides and having a nucleotide sequence that yeast-derived Flp recombinase recognizes and catalyzes the homologous recombination thereof.

The term "homologous" refers to the fact that the aforementioned linear DNA has homology to the extent that homologous recombination takes place in a target region on a chromosomal DNA. Specifically, it refers to 80% or more, preferably 90% or more, more preferably 95% or more, and further preferably 100% homology.

Homology of the nucleotide sequences can be determined using the aforementioned programs such as BLAST or FASTA.

The aforementioned linear DNA can be prepared by PCR. Also, DNA containing the linear DNA can be constructed on the plasmid and the linear DNA of interest can then be obtained by restriction enzyme treatment.

Deletion, substitution, or addition of nucleotide(s) can be introduced into chromosomal DNAs of microorganisms by any of the following methods 1 to 4:
Method 1: a method wherein the linear DNA (a) or (d) is introduced into a host microorganism, and a transformant, into which such linear DNA has been integrated into a chromosomal DNA by homologous recombination, is selected using drug resistance as an indicator;
Method 2: a method wherein the linear DNA (b) is introduced into the transformant obtained by the above method 1, and the drug-resistant gene integrated into chromosomal DNA by this method is deleted so as to substitute or delete a region on a chromosomal DNA of a microorganism;
Method 3:
   [1] introducing the linear DNA (c) into a host microorganism and selecting a transformant into which the linear DNA has been integrated into chromosomal DNA by homologous recombination using drug resistance as an indicator,
   [2] synthesizing a DNA obtained by ligating DNAs homologous to DNAs located at both outside regions of a target region of substitution or deletion on a chromosomal DNA in the same direction on the chromosomal DNA and introducing the synthesized product into the transformant obtained in [1] above, and
   [3] culturing transformants subjected to the procedure [2] under conditions where the genes that can be used for negative selection are expressed, and selecting strains that can grow in the culture condition as strains in which the drug-resistant genes and genes that can be used for negative selection have been deleted from the chromosomal DNA; and
Method 4:
   [1] introducing the linear DNA (d) into a host microorganism and selecting a transformant into which the linear DNA has been integrated into chromosomal DNA by homologous recombination using drug resistance as an indicator, and
   [2] introducing Flp recombinase gene-expressing plasmid into the transformant obtained in the above [1] to express the Flp recombinase gene, and obtaining strains sensitive to the drug used in the above[1].

A method of introducing a linear DNA into a host microorganism that is employed in the above methods can be any method as long as the DNA can be introduced into the microorganism. Examples thereof include a method involving the use of calcium ions [Proc. Natl. Acad. Sci., U.S.A., 69, 2110, 1972], the protoplast method [Japanese Published and Unexamined Patent Application No. 2483942/88(1988)], and electroporation [Nucleic Acids Res., 16, 6127, 1988].

By using the linear DNA comprising at a site around the center thereof any gene to be inserted into a chromosomal DNA as the linear DNA that is used in method 2 or method 3 [2], drug-resistant genes or the like can be deleted, and any genes can be simultaneously introduced into the chromosomal DNA.

According to the methods 2 to 4, foreign genes, such as drug-resistant genes and genes that can be used for negative selection, do not remain in the chromosomal DNA of the transformant which is finally obtained. With the utilization of such methods, accordingly, the same drug-resistant genes and genes that can be used for negative selection may be used, and the procedures of such methods are repeated. This enables the easy production of microorganisms having deletion, substitution or addition of nucleotide(s) at two or more different regions on a chromosomal DNA.

### 3. A process of the present invention for producing a useful substance using the microorganism.

The microorganisms 2 above in the above 2 are cultured in a medium to form and accumulate an amino acid in the culture, and the amino acid are recovered from the culture. Thus, such amino acids can be produced. The microorganisms can be cultured in a medium in accordance with a conventional method for culturing microorganisms.

Specifically, a medium for culturing such microorganisms may be natural or synthetic medium as long as it contains carbon sources, nitrogen sources, inorganic salts, etc. assimilable by the microorganisms and the microorganisms can be efficiently cultured therein.

Any carbon sources assimilable by the microorganisms can be used. Examples thereof include: carbohydrates such as glucose, fructose, sucrose, molasses containing any of such substances, starch, and starch hydrolysate; organic acids such as acetic acid and propionic acid; and alcohols such as ethanol and propanol.

Examples of nitrogen sources include: ammonia; ammonium salts of inorganic or organic acids such as ammonium chloride, ammonium sulfate, ammonium acetate, and ammonium phosphate; other nitrogen-containing compounds; peptone; meat extract; yeast extract; corn steep liquor; casein hydrolysate; soybean cake and hydrolysate of soybean cake; and various fermentation microorganisms and digests thereof.

Examples of inorganic salts include: monopotassium phosphate, dipotassium phosphate, magnesium phosphate, magnesium sulfate, sodium chloride, iron(I) sulfate, manganese sulfate, copper sulfate, and calcium carbonate.

Usually, the culture is carried out under aerobic conditions such as shaking culture or submerged aeration agitation culture. Culture temperature is preferably 15 to 40°C, and culture time is generally 5 hours to 7 days. During the culture, the pH is maintained at 3.0 to 9.0. The pH is adjusted with an inorganic or organic acid, an alkali solution, urea, calcium carbonate, ammonia, or the like.

During the culture, an antibiotic such as ampicillin or tetracycline may be added to the medium, if necessary.

When a microorganism transformed with an expression vector containing an inducible promoter as a promoter is cultured, an inducer may be added to the medium, if necessary. For example, when a microorganism transformed with an expression vector containing *lac* promoter is cultured, isopropyl-β-D-thiogalactopyranoside or the like may be added to the medium. When a microorganism transformed with an expression vector containing *trp* promoter is cultured, indoleacrylic acid or the like may be added to the medium.

Useful substances formed and accumulated in the culture product can be obtained by a conventional method involving the use of active carbon or ion-exchange resins or extraction using an organic solvent, crystallization, thin-layer chromatography, or high-performance liquid chromatography.

Examples are provided below, although the present invention is not limited to these Examples.

### Example 1

### Construction of useful substance-producing strains that lack ihfA gene

### (1) Construction of E. coli capable of homologous recombination with a linear DNA on its chromosomal DNA

### (i) Production of E. coli W3110red strains

The P1 phages were prepared from the *E. coli* KM22 strains [Gene, 246, 321-330, 2000] capable of homologous recombination with a linear DNA on a chromosomal DNA.

The *E. coli* KM 22 strains were cultured in LB liquid medium containing 20 mg/l of kanamycin (10 g/l of Bacto tryptone (Difco), 5 g/l of Bacto yeast extract (Difco), 5 g/l of sodium chloride, and 1 ml/l of 1 mol/l sodium hydroxide) at 30°C overnight. Thereafter, 0.5 ml of the culture solution, 1.4 ml of fresh LB liquid medium, and 100 µl of 0.1 mol/l calcium chloride solution were mixed.

The resulting mixture was subjected to shaking culture at 30°C for 5 to 6 hours, and 400 µl of the mixture was transferred to a sterilized tube. A P1 phage stock solution (2 µl) was added thereto, the mixture was heated at 37°C for 10 minutes, 3 ml of a soft agar solution maintained at 50°C (10 g/l of Bacto tryptone, 5 g/l of Bacto yeast extract, 5 mmol/l of calcium chloride, 1 ml/l of 1 mol/l sodium hydroxide, and 3 g/l of agar) was added thereto, the resultant was thoroughly agitated, and the resultant was then applied to a Ca-LB agar medium plate (10 g/l of Bacto tryptone, 5 g/l of Bacto yeast extract, 5 mmol/l of calcium chloride, 1 ml/l of 1 mol/l sodium hydroxide, and 15 g/l of agar; plate diameter: 15 cm).

The plate was subjected to culture at 37°C for 7 hours, a soft agar portion was finely ground using a spreader and recovered, and the resultant was centrifuged at 1,500 g at 4°C for 10 minutes. Chloroform (100 µl) was added to 1.5 ml of the supernatant, and the mixture was stored at 4°C overnight. On the following day, the solution was centrifuged at 15,000 g for 2 minutes, and the supernatant was stored at 4°C as a phage stock.

Subsequently, the obtained phage was used to transform the *E. coli* W3110 strain. The culture solution (200 µl) of the *E. coli* W3110 strains that had been cultured in Ca-LB liquid medium at 30°C for 4 hours was separated, and 1 µl of phage stock was added. The mixture was incubated at 37°C for 10 minutes, 5 ml of LB liquid medium and 200 µl of 1 mol/l sodium citrate solution were added, and the mixture was agitated. The resultant was centrifuged at 1,500 g at 25°C for 10 minutes, the supernatant was discarded, 1 ml of LB liquid medium and 10 µl of 1 mol/l sodium citrate solution were added to the precipitated cells, and the resultant was incubated at 30°C for 2 hours. The solution (100 µl) was applied to an agar plate medium, Antibiotic Medium 3, containing 20 mg/l of kanamycin, and the plate was subjected to culture at 30°C overnight. Twenty-four colonies that had appeared for each deletion-introduced strain were applied to an agar plate medium, Antibiotic Medium 3, containing 20 mg/l kanamycin (0.15% meat extract, 0.15% yeast extract, 0.5% peptone, 0.1% glucose, 0.35% sodium chloride, 0.132% dibasic potassium phosphate, and 1.5% agar; pH 7), the drug sensitivity thereof was confirmed, and kanamycin-resistant strains were selected. Thus, *E. coli* W3110red strains into which the chromosomal DNA region (Δ(recC ptr recB recD)::Plac-red kan) capable of homologous recombination with a linear DNA on chromosomal DNA had been transduced, were obtained.

### (ii) Construction of E. coli W3110red_tet strains

PCR was carried out using the chromosomal DNA of the *E. coli* W3110red strain as a template and, as a primer set, DNA comprising the nucleotide sequences as shown in SEQ ID NOs: 6 and 7. PCR was carried out by using LA-Taq (Takara Bio Inc.), preparing 100 µl of a reaction solution containing 10 ng of the chromosomal DNA and 50 pmol of each primer DNA in accordance with the instructions included with LA-Taq, incubating at 94°C for 2 minutes, repeating a cycle of 94°C for 15 seconds, 55°C for 20 seconds, and 68°C for 3 minutes 30 times, and then incubating at 72°C for 10 minutes.

Agarose gel electrophoresis was carried out to confirm that the DNA fragment of interest had been amplified, and the DNA fragment was recovered from the gel, followed by purification.

Subsequently, PCR was carried out by using the chromosomal DNA of the *E*. *coli* CGSC7465 strains having Tn10 transposons on chromosomal DNA (available from the *E. coli* Genetic Stock Center managed by Yale University, U.S.A.) as a template and, as a primer set, DNAs comprising the nucleotide sequences as shown in SEQ ID NOs: 8 and 9. PCR was carried out by using LA-Taq, preparing 100 µl of a reaction solution containing 10 ng of the chromosomal DNA and 50 pmol of each primer DNA in accordance with the instructions included with LA-Taq, incubating at 94°C for 2 minutes, repeating a cycle of 94°C for 15 seconds, 55°C for 20 seconds, and 68°C for 90 seconds 30 times, and then incubating at 72°C for 10 minutes.

Agarose gel electrophoresis was carried out to confirm that the DNA fragment of interest had been amplified, and the DNA fragment of interest was recovered from the gel, followed by purification.

Subsequently, PCR was carried out using the chromosomal DNA of the *E. coli* W3110red strains as a template and, as a primer set, DNAs comprising the nucleotide sequences as shown in SEQ ID NOs: 10 and 11. PCR was carried out by using LA-Taq, preparing 100 µl of a reaction solution containing 10 ng of the chromosomal DNA and 50 pmol of each primer DNA in accordance with the instructions included with LA-Taq, incubating at 94°C for 2 minutes, repeating a cycle of 94°C for 15 seconds, 55°C for 20 seconds, and 68°C for 3 minutes 30 times, and then incubating at 72°C for 10 minutes.

Agarose gel electrophoresis was carried out to confirm that the DNA fragment of interest had been amplified, and the DNA fragment was recovered from the gel, followed by purification.

Subsequently, PCR was carried out by using the three DNA fragments obtained by the above PCR procedures as templates and, as a primer set, DNAs comprising the nucleotide sequences as shown in SEQ ID NOs: 12 and 13. PCR was carried out using LA-Taq, preparing 100 µl of a reaction solution containing 10 ng each of the amplified DNA fragment and 50 pmol of each primer DNA in accordance with the instructions included with LA-Taq, incubating at 94°C for 2 minutes, repeating a cycle of 94°C for 15 seconds, 55°C for 20 seconds, and 68°C for 3 minutes 30 times, and then incubating at 72°C for 10 minutes.

It was confirmed by agarose gel electrophoresis that a DNA fragment having at both ends a sequence homologous to the kanamycin-resistant gene and at the center a tetracycline-resistant gene was amplified by the above PCR procedures. Thereafter, the DNA fragment (hereafter referred to as a *tet* gene fragment) was recovered from the gel and then purified.

Subsequently, competent cells of *E. coli* W3110 red strains were prepared in accordance with the method described in Gene, 246, 321-330, 2000, and the obtained competent cells were transformed with 1 µg of *tet* gene fragment. Transformation was carried out by electroporation in a 0.1 cm cuvette (BioRad) at 1.8 kV and 25 µF.

The transformed cells were cultured using 1 ml of SOC liquid medium [20 g/l of Bacto tryptone (Difco), 5 g/l of Bacto yeast extract (Difco), 0.5 g/l of sodium chloride, 0.2 ml/l of 5 mol/l NaOH, 10 ml/l of 1 mol/l magnesium chloride, 10 ml/l of 1 mol/l magnesium sulfate, and 10 ml/l of 2M glucose] containing 2 mmol/l IPTG at 30°C for 3 hours. Thereafter, the culture was spread on an LB agar plate (containing 1.5% agar in LB liquid medium) containing 20 µg/ml of tetracycline, and the plate was incubated at 37°C overnight.

The grown strain was confirmed to be a strain in which kanamycin-resistant genes on the chromosomal DNA of *E. coli* W3110red strains had been substituted with tetracycline-resistant genes, and this strain was designated as *E. coli* W3110red_tet strains.

### (2) Construction of proline-producing strains

The ability to produce proline was imparted to the *E. coli* W3110red strains obtained in (1) above in the following manner.

### (i) Construction of putA gene-deficient strains

PCR was carried out using pHSG398 plasmid DNA (purchased from Takara Bio Inc.) as a template and, as a primer set, DNAs comprising the nucleotide sequences as shown in SEQ ID NOs: 14 and 15. Thus, a DNA fragment containing a chloramphenicol-resistant gene was amplified. PCR was carried out by using EX-Taq (Takara Bio Inc.), preparing 100 µl of a reaction solution containing 20 ng of purified plasmid DNA and 50 pmol of each primer DNA in accordance with the instructions included with EX-Taq, incubating at 95°C for 1 minute, repeating a cycle of 94°C for 30 seconds, 64°C for 30 seconds, and 72°C for 1 minute 30 times, and then incubating at 72°C for 3 minutes. The amplified DNA fragment was purified using the QIA quick PCR Purification Kit (Qiagen) to obtain 30 µl of a DNA solution.

Subsequently, 10 U of each of the restriction enzymes *PstI* and *BglI* was added to the DNA solution to digest a trace amount of contaminating plasmid pHSG398, the mixture was incubated at 37°C for 2 hours, and the amplified DNA fragment was purified using the QIA quick PCR Purification Kit to obtain 30 µl of a DNA solution.

PCR was then carried out by using 0.1 µl of the DNA solution as a template and, as a primer set, DNAs comprising the nucleotide sequences as shown in SEQ ID NOs: 16 and 17 to amplify a DNA fragment having at its center the chloramphenicol-resistant gene and at both of its ends DNA comprising the nucleotide sequence as shown in SEQ ID NO: 18 (DNA at the 5' end of the *putA* gene) and DNA comprising the nucleotide sequence as shown in SEQ ID NO: 19 (DNA at the 3' end of the *putA* gene).

PCR was carried out by using EX-Taq, preparing 100 µl of a reaction solution containing 10 ng of purified template DNA and 50 pmol of each primer DNA in accordance with the instructions included with EX-Taq, incubating at 95°C for 1 minute, repeating a cycle of 94°C for 30 seconds, 51°C for 30 seconds, and 72°C for 1 minute 30 times, and then incubating at 72°C for 3 minutes. Thus, the amplified DNA fragment was purified in the same manner as described above.

Subsequently, the *E. coli* W3110 red strains were transformed by using 1 µg of the DNA. Transformation was carried out under the same conditions as with the case of the electroporation of above (1).

The transformed cells were cultured in 1 ml of SOC liquid medium containing 2 mmol/l of IPTG at 30°C for 3 hours, the culture was spread on an LB agar plate (containing 1.5% agar in LB liquid medium; hereafter abbreviated as "LB cm agar medium plate") containing 50 µg/ml chloramphenicol, and the plate was then incubated at 37°C overnight.

The grown strain was confirmed to be a strain in which *putA* gene on the chromosomal DNA of the *E. coli* W3110red strain had been substituted with chloramphenicol-resistant gene, and this strain was designated as the *E. coli* W3110redΔ*putA::cat* strain.

### (ii) Construction of proBA operon-deficient strains

PCR was carried out by using the plasmid pFastBAC dual (purchased from Invitrogen) as a template and, as a primer set, DNAs comprising the nucleotide sequences as shown in SEQ ID NOs: 20 and 21. Thus, a DNA fragment containing a gentamicin-resistant gene was amplified. PCR was carried out by using LA-Taq, preparing 25 µl of a reaction solution containing 10 ng of purified plasmid DNA and 20 pmol of each primer DNA in accordance with the instructions included with LA-Taq, incubating at 94°C for 3 minutes, repeating a cycle of 94°C for 15 seconds, 55°C for 20 seconds, and 68°C for 1 minute 30 times, and then incubating at 72°C for 10 minutes.

After the completion of the reaction, the amplified DNA fragment was purified in the same manner as described above to obtain 50 µl of a DNA solution. PCR was then carried out using the amplified DNA fragment obtained by the above PCR as a template and, as a primer set, DNAs comprising the nucleotide sequences as shown in SEQ ID NOs: 22 and 23 to amplify a DNA fragment having at its center the gentamicin-resistant gene and at both of its ends DNA comprising the nucleotide sequence as shown in SEQ ID NO: 24 (DNA at the 5' end of the *proBA* operon) and DNA comprising the nucleotide sequence as shown in SEQ ID NO: 25 (DNA at the 3' end of the *proBA* operon).

PCR was carried out by using LA-Taq, preparing 25. µl of a reaction solution containing 10 ng of the amplified DNA fragment and 20 pmol of each primer DNA in accordance with the instructions included with LA-Taq, heating at 94°C for 3 minutes, repeating a cycle of 94°C for 15 seconds, 55°C for 20 seconds, and 68°C for 1 minute 30 times, and then heating at 72°C for 10 minutes. The amplified DNA fragment (hereafter referred to as the "amplified DNA fragment A") was purified in the same manner as described above.

PCR was carried out by using the chromosomal DNA of the *E. coli* W3110 red strains as a template and, as a primer set, DNAs comprising the nucleotide sequences as shown in SEQ ID NOs: 26 and 27 to amplify a DNA fragment of approximately 1 kbp located in the 5'-upstream region of the *proBA* operon. Also, PCR was carried out using, as a primer set, DNAs comprising the nucleotide sequences as shown in SEQ ID NOs: 28 and 29 to amplify a DNA fragment of approximately 1 kbp located in the 3'-downstream region of the *proBA* operon.

PCR was carried out by preparing 25 µl of a reaction solution containing 10 ng of the chromosomal DNA and 20 pmol of each primer DNA in accordance with the instructions included with LA-Taq, incubating at 94°C for 3 minutes, repeating a cycle of 94°C for 15 seconds, 55°C for 20 seconds, and 68°C for 1 minute 30 times, and then incubating at 72°C for 10 minutes. The amplified DNA fragments (amplified DNA fragments B and C) were purified in the same manner as described above.

Subsequently, PCR was carried out using the amplified DNA fragments A, B, and C obtained above as templates and, as a primer set, DNAs comprising the nucleotide sequences as shown in SEQ ID NOs: 26 and 29. PCR was carried out by using LA-Taq, preparing 25 µl of a reaction solution containing 10 ng each of the amplified DNA fragments A, B, and C and 20 pmol of each primer DNA in accordance with the instructions included with LA-Taq, incubating at 94°C for 3 minutes, repeating a cycle of 94°C for 15 seconds, 55°C for 20 seconds, and 68°C for 3 minutes 30 times, and then incubating at 72°C for 10 minutes.

Agarose gel electrophoresis was carried out to confirm that a DNA fragment, which is a ligation product of DNA fragments A, B, and C, had been amplified. Thereafter, the DNA fragment was recovered from gel and then purified. The DNA fragment comprised at both ends sequences homologous to 1-kbp-DNA located in the 5'-upstream and 3'-downstream regions of the *proBA* operon on the chromosomal DNA and at the center the gentamicin-resistant gene.

Subsequently, the *E. coli* W3110redΔ*putA::cat* strain was transformed using 1 µg of the above DNA fragment. Transformation was carried out by electroporation, as with the case of transformation of the *E. coli* W3110red strain. As a medium for selecting transformants, an LB agar plate containing 5 µg/ml of gentamicin was used. The strain that had grown on the agar plate was confirmed to be a strain in which the *proBA* operon on the chromosomal DNA of the *E. coli* W3110redΔ*putA::cat* strain had been substituted with gentamicin-resistant gene, and this strain was designated as *E. coli* PK strain.

The *E. coli* PK strain was spread on the M9 minimal agar medium and on the M9 minimal agar medium containing 8 mg/l of proline, and the strains were cultured at 30°C overnight. As a result, it was confirmed that the *E. coli* PK strain grew in a proline-containing minimal medium but did not grow in a proline-free minimal medium.

### (iii) Construction of mutant proBA operon-introduced strains

PCR was carried out using the chromosomal DNA of the *E. coli* W3110red strain as a template and, as a primer set, DNAs comprising the nucleotide sequences as shown in SEQ ID NOs: 26 and 29 to amplify a 4-kbp DNA fragment containing the *proBA* operon. PCR was carried out using LA-Taq, preparing 25 µl of a reaction solution containing 10 ng of the chromosomal DNA and 20 pmol of each primer DNA in accordance with the instructions included with LA-Taq, incubating at 94°C for 3 minutes, repeating a cycle of 94°C for 15 seconds, 55°C for 20 seconds, and 68°C for 4 minutes 30 times, and then incubating at 72°C for 10 minutes.

Agarose gel electrophoresis was carried out to confirm that the DNA fragment of interest had been amplified. Thereafter, the DNA fragment of interest was recovered from gel, purified, and then cloned into the plasmid pPCR-Script Amp using the PCR-Script Cloning Kit (Qiagen). Thus, the plasmid pPCR proBA was constructed.

Subsequently, point mutation proB74 [Gene, 64, 199-205, 1988] was introduced into the *proB* gene of pPCR proBA using the QuickChange Kit (Qiagen) in accordance with the instructions included with the kit to produce a plasmid pPCR proB74. SEQ ID NO: 30 shows the nucleotide sequence of primer DNA used for introduction of point mutation.

The plasmid pPCR proB74 was cleaved using the restriction enzyme *BsrGI*, a DNA fragment containing the mutant *proBA* operon was separated by agarose electrophoresis, and the separated fragment was recovered and purified from the gel. The *E. coli* PK strain was transformed using 2 µg of the DNA fragment. Transformation was carried out by electroporation, as with the case of the aforementioned *E. coli* W3110red strain. The transformants were selected using the M9 minimal agar medium, and strains that did not show proline requirement were selected to obtain the *E. coli* PKB74 strain capable of producing proline.

### (3) Construction of proline-producing strains that lack ihfA gene

PCR was carried out by using the chromosomal DNA of the *E. coli* W3110red strain as a template and, as a primer set, DNAs comprising the nucleotide sequences as shown in SEQ ID NOs: 31 and 32, the 25-bp region at the 3'-end of each of which being designed to hybridize to both ends of the kanamycin-resistant genes on the chromosomal DNA of the W3110red strains. PCR was carried out by using LA-Taq, preparing 25 µl of a reaction solution containing 10 ng of the chromosomal DNA fragment and 20 pmol of each primer DNA in accordance with the instructions included with LA-Taq, incubating at 94°C for 2 minutes, repeating a cycle of 94°C for 15 seconds, 55°C for 20 seconds, and 68°C for 1 minute 30 times, and then incubating at 72°C for 10 minutes.

The amplified DNA fragment comprising the nucleotide sequence as shown in SEQ ID NO: 33 in the 5'-upstream region of the kanamycin-resistant gene and the nucleotide sequence as shown in SEQ ID NO: 34 in the 3'-downstream region thereof was purified in the manner as described above.

PCR was then carried out by using the DNA fragment as a template and, as a primer set, DNAs comprising the nucleotide sequences as shown in SEQ ID NOs: 35 and 36. DNA comprising the nucleotide sequence as shown in SEQ ID NO: 35 has at the 5' end a 40-bp nucleotide sequence homologous to a sequence in the vicinity of the 5'-end region of the *ihfA* gene, and at the 3' end it has DNA comprising the nucleotide sequence as shown in SEQ ID NO: 33. DNA comprising the nucleotide sequence as shown in SEQ ID NO: 36 has at the 5' end a 40-bp nucleotide sequence homologous to a sequence in the vicinity of the 3'-end region of the *ihfA* gene, and at the 3' end it has DNA comprising a 25-bp nucleotide sequence that hybridizes to the nucleotide sequence as shown in SEQ ID NO: 34.

PCR was carried out by using LA-Taq, preparing 25 µl of a reaction solution containing 10 ng of the amplified DNA fragment and 20 pmol of each primer DNA in accordance with the instructions included with LA-Taq, incubating at 94°C for 2 minutes, repeating a cycle of 94°C for 15 seconds, 55°C for 20 seconds, and 68°C for 1 minute 30 times, and then incubating at 72°C for 10 minutes.

It was confirmed that a DNA fragment which comprises kanamycin-resistant gene (km gene) having at both ends regions homologous to 40-bp regions at both ends of the *ihfA* gene, had been amplified. The DNA fragment of interest was purified in the same manner as described above.

The *E. coli* PKB74_tet strain was transformed using 1 µg of the DNA fragment. Transformation was carried out by electroporation as with the case of the *E. coli* W3110red strain described above. The transformed cells were cultured in the same manner as described above with the use of SOC medium, the culture liquid was spread on an LB agar medium containing 50 µg/ml of kanamycin, and the culture was then incubated at 30°C overnight.

The grown strain was confirmed to lack the *ihfA* gene on the chromosomal DNA of the *E. coli* PKB74_tet strains, and this strain was designated as *E. coli* PKB74_tetAihfA strain.

### Example 2

### Construction of useful substances with the use of ihfA gene-deficient strains

The *E. coli* PKB74_tetAihfA strain and the control strain, i.e., the *E. coli* PKB74_tet strain, were inoculated into test tubes each containing 5 ml of LB liquid medium and subjected to shaking culture at 30°C for 20 hours to obtain cultures. The cultures (500 µl each) were inoculated into 300-ml flasks each containing 50 ml of Med7 liquid medium (3% glucose, 0.8% peptone, 0.1% monobasic potassium phosphate, 0.05% magnesium sulfate heptahydrate, 0.002% calcium chloride, 1% calcium carbonate, 1% ammonium sulfate, 0.2% sodium chloride, and 0.03% iron sulfate) and then subjected to shaking culture at 30°C.

Parts of the cultures were sampled 30, 33, and 36 hours after the initiation of culture and then subjected to centrifugation. The supernatants were diluted 1,000-fold, and the amounts of proline that had accumulated in the cultures were measured by using the direct amino acid analysis system (Dxa-500, Dionex). The results are shown in Table 1.

### [Table 1]

**Table 1**

| Strains | Proline (g/l) | | |
|---|---|---|---|
| | 30 hours later | 33 hours later | 36 hours later |
| E. coli *PKB74_tet*Δ*ihfA* | 2.5 | 2.9 | 3.5 |
| E. coli *PKB74_tet* | 2.2 | 2.3 | 2.6 |

As shown in Table 1, the ability to produce proline of the *E. coli* PKB74_tetΔihfA strain, which is the *ihfA* gene-deficient strain constructed from a proline-producing *E. coli* PKB74_tet strain, was found to be superior to that of the *E. coli* PKB74_tet strain.

### Example 3

### Analysis of gene expression profile of E. coli PKB74_tetΔihfA strain

Gene expression profiles of the *E. coli* PKB74_tetΔihfA strain and its parent strain, i.e., the *E. coli* PKB74_tet strain, were analyzed in the following manner.

Parts of the cultures of each of the strains 28 hours after the initiation of culture that had been conducted in Example 2 were sampled, and transcription patterns thereof were compared using DNA slide arrays in accordance with the method of Ohshima et al. [Mol. Microbiol., 45, 673-695, 2002]. As DNA slide arrays, arrays for *E. coli* available from Takara Bio Inc. were purchased and used.

On the basis of the mRNA level in the *E. coli* PKB74_tet strains, the expression levels of genes of the *E. coli* PKB74_tetΔihfA strain were analyzed. As a result, the expression level of the *pfkB* gene, which are the structural gene of 6-phosphofructokinase in the rate-determining steps in the glycolytic pathway, was found to have increased to 12 times the aforementioned mRNA level. Also, the expression levels of *acnA* genes and *sucAB* genes were found to have increased to 8 times and to 3 times, respectively, among the enzyme structural genes that constitute the TCA cycle.

In contrast, the expression levels of proline biosynthetic genes, *proBA* gene and *proC* gene, were equivalent to those of the *E. coli* PKB74_tet strains.

Thus, it was demonstrated that the ability of the *ihfA* gene-deficient strain for proline production was caused by the enhanced activity of central metabolic systems, such as the glycolytic pathway and the TCA cycle. Accordingly, it was also demonstrated that the *ihfA* gene-deficient strains would improve the ability to produce various useful substances generated via the glycolytic pathway and the TCA cycle, such as proteins, peptides, nucleic acids, vitamins, saccharides, organic acids, and lipids, in addition to amino acids such as proline.

### Example 4

### Construction of ihfA gene-deficient strains

The *E. coli* PKB74_tetΔihfA strain prepared in Example 1(3) was infected with P1 phages in the manner described in Example 1(1), and a phage stock for transformation was recovered therefrom. The *E. coli* W3110 strain was infected with the obtained phage stock for transformation and kanamycin-resistant strain was separated by the method described in Example 1(1). The kanamycin-resistant strain was a strain in which the *ihfA* gene of the *E. coli* W3110 strain had been deleted by substitution with kanamycin-resistant gene. This strain was designated as the *E. coli* W3110Δ*ihfA* strain.

### Example 5

### Production of useful substances with the use of ihfA gene-deficient strains

The *E. coli* W3110Δ*ihfA* strain and its parent strain, i.e., the *E. coli* W3110 strain, were inoculated into test tubes each containing 5 ml of LB liquid medium and subjected to shaking culture at 30°C for 20 hours to obtain cultures. The cultures (300 µl each) were inoculated into 300-ml flasks each containing 30 ml of Med7 liquid medium (3% glucose, 0.8% peptone, 0.1% monobasic potassium phosphate, 0.05% magnesium sulfate heptahydrate, 0.002% calcium chloride, 1% calcium carbonate, 1% ammonium sulfate, 0.2% sodium chloride, and 0.03% iron sulfate) and then subjected to shaking culture at 30°C.

Parts of the cultures were sampled 18, 30, and 42 hours after the initiation of culture and then subjected to centrifugation. The supernatants were diluted 1,000-fold, and the amounts of glutamic acid that had accumulated in the cultures were measured using the direct amino acid analysis system (Dxa-500, Dionex). The results are shown in Table 2.

**Table 2**

| Strains | Glutamic acid (g/l) | | |
|---|---|---|---|
| | 30 hours later | 33 hours later | 36 hours later |
| E. coli E3110ΔihfA | 0.7 | 1.3 | 2.1 |
| *E. coli W3110* | 0.3 | 0.8 | 1.0 |

As shown in Table 2, the ability of the *ihfA* gene-deficient strain for glutamic acid production was found to be approximately twice that of the *E. coli* E3110 strain.

### Example 6

### Construction of ihfB gene-deficient strains

PCR was carried out by using the chromosomal DNA of the *E. coli* W3110red strain as a template and, as a primer set, DNAs comprising the nucleotide sequences as shown in SEQ ID NOs: 31 and 32, a 25-mer region at the 3'-end of each of which being designed to hybridize to both ends of kanamycin-resistant genes on chromosomal DNA of the *E. coli* W3110red strain.

PCR was carried out using LA-Taq, preparing 25 µl of a reaction solution containing 10 ng of the chromosomal DNA fragment and 20 pmol of each primer DNA in accordance with the instructions included with LA-Taq, incubating at 94°C for 2 minutes, repeating a cycle of 94°C for 15 seconds, 55°C for 20 seconds, and 68°C for 1 minute 30 times, and then incubating at 72°C for 10 minutes.

By this PCR procedure, it was confirmed that a DNA fragment comprising the nucleotide sequence as shown in SEQ ID NO: 33 in the 5'-upstream region of the kanamycin-resistant gene and the nucleotide sequence as shown in SEQ ID NO: 34 in the 3'-downstream region had been amplified. The DNA fragment was purified in the same manner as described above.

Subsequently, PCR was carried out by using the purified DNA fragment as a template and, as a primer set, DNA comprising the nucleotide sequence as shown in SEQ ID NO: 37, which has at the 5'-end a 40-mer nucleotide sequence identical to the 5'-end region of the *ihfB* structural gene, and has at the 3'-end a 25-mer nucleotide sequence as shown in SEQ ID NO: 33, and DNA comprising the nucleotide sequence as shown in SEQ ID NO: 38, which has at the 5'-end a 40-mer nucleotide sequence that hybridizes to a 3'-end region of the *ihfB* structural genes, and has at the 3'-end a 25-mer nucleotide sequence that hybridizes to the nucleotide sequence as shown in SEQ ID NO: 34.

PCR was carried out by using LA-Taq, preparing 25 µl of a reaction solution containing 10 ng of the amplified DNA fragment and 20 pmol of each primer DNA in accordance with the instructions included with LA-Taq, incubating at 94°C for 2 minutes, repeating a cycle of 94°C for 15 seconds, 55°C for 20 seconds, and 68°C for 1 minute 30 times, and then incubating at 72°C for 10 minutes.

By this PCR procedure, it was confirmed that a DNA fragment containing a kanamycin-resistant gene (km gene) having at both of its ends the nucleotide sequence identical to a 40-bp region at both ends of the *ihfB* structural gene had been amplified. The DNA fragment was purified in the same manner as described above.

The *E. coli* PKB74_tet strain was transformed using 1 µg of the above DNA. Transformation was carried out via electroporation, as with the case of the *E. coli* W3110red strain described above. The transformed cells were cultured in the same manner as described above using SOC medium, the culture was spread on an LB agar plate containing 50 µg/ml of kanamycin, and the plate was incubated at 30°C overnight.

The grown strains were found to lack the *ihfB* gene on the chromosomal DNA of the *E. coli* PKB74_tet strain. Thus, the strain was designated as the *E. coli* PKB74_tetΔihfB strain.

### Example 7

### Construction of useful substances with the use of ihfB gene-deficient strains

In the same manner as in Example 2, the *E. coli* PKB74_tetΔihfB strain and its control strain, *E. coli* PKB74_tet, were cultured, and the amount of L-proline accumulated in the culture was measured. The results are shown in Table 3.

**Table 3**

| Strains | Proline (g/l) | | |
|---|---|---|---|
| | 16 hours later | 27 hours later | 40 hours later |
| E. coli *PKB74_tetΔihfB* | 0.9 | 2.5 | 5.1 |
| E. coli *PKB74_tet* | 0.7 | 2.3 | 2.7 |

As shown in Table 3, the ability to produce proline of the *E. coli* PKB74_tetΔihfB strain, which is the *ihfB* gene-deficient strain produced from a proline-producing *E. coli* PKB74_tet strain, was found to be superior to that of the *E. coli* PKB74_tet strain.

Accordingly, the *ihfB* gene-deficient strain was found to exhibit improved efficiency for producing a useful substance, as with the case of the *ihfA* gene-deficient strain.

### Free text of the Sequence Listing

| | |
|---|---|
| SEQ ID NO:5 | Explanation of Artificial sequence: synthesized DNA |
| SEQ ID NO:6 | Explanation of Artificial sequence: synthesized DNA |
| SEQ ID NO:7 | Explanation of Artificial sequence: synthesized DNA |
| SEQ ID NO:8 | Explanation of Artificial sequence: synthesized DNA |
| SEQ ID NO:9 | Explanation of Artificial sequence: synthesized DNA |
| SEQ ID NO: 10 | Explanation of Artificial sequence: synthesized DNA |
| SEQ ID NO:11 | Explanation of Artificial sequence: synthesized DNA |
| SEQ ID NO: 12 | Explanation of Artificial sequence: synthesized DNA |
| SEQ ID NO: 13 | Explanation of Artificial sequence: synthesized DNA |
| SEQ ID NO:14 | Explanation of Artificial sequence: synthesized DNA |
| SEQ ID NO: 15 | Explanation of Artificial sequence: synthesized DNA |
| SEQ ID NO: 16 | Explanation of Artificial sequence: synthesized DNA |
| SEQ ID NO: 17 | Explanation of Artificial sequence: synthesized DNA |
| SEQ ID NO: 18 | Explanation of Artificial sequence: synthesized DNA |
| SEQ ID NO:19 | Explanation of Artificial sequence: synthesized DNA |
| SEQ ID NO:20 | Explanation of Artificial sequence: synthesized DNA |
| SEQ ID NO:21 | Explanation of Artificial sequence: synthesized DNA |
| SEQ ID NO:22 | Explanation of Artificial sequence: synthesized DNA |
| SEQ ID NO:23 | Explanation of Artificial sequence: synthesized DNA |
| SEQ ID NO:24 | Explanation of Artificial sequence: synthesized DNA |
| SEQ ID NO:25 | Explanation of Artificial sequence: synthesized DNA |
| SEQ ID NO:26 | Explanation of Artificial sequence: synthesized DNA |
| SEQ ID NO:27 | Explanation of Artificial sequence: synthesized DNA |
| SEQ ID NO:28 | Explanation of Artificial sequence: synthesized DNA |
| SEQ ID NO:29 | Explanation of Artificial sequence: synthesized DNA |
| SEQ ID NO:30 | Explanation of Artificial sequence: synthesized DNA |
| SEQ ID NO:31 | Explanation of Artificial sequence: synthesized DNA |
| SEQ ID NO:32 | Explanation of Artificial sequence: synthesized DNA |
| SEQ ID NO:33 | Explanation of Artificial sequence: synthesized DNA |
| SEQ ID NO:34 | Explanation of Artificial sequence: synthesized DNA |
| SEQ ID NO:35 | Explanation of Artificial sequence: synthesized DNA |
| SEQ ID NO:36 | Explanation of Artificial sequence: synthesized DNA |
| SEQ ID NO:37 | Explanation of Artificial sequence: synthesized DNA |
| SEQ ID NO:38 | Explanation of Artificial sequence: synthesized DNA |

## Claims

1. A process for producing an amino acid which comprises culturing a microorganism in a medium so as to form and accumulate the amino acid in the culture product and recovering the amino acid from the culture product, wherein the microorganism belongs to the genus *Erwinia, Serratia, Salmonella, Escherichia, Proteus, Pseudomonas*, or *Xanthomonas and*
wherein the microorganism comprises chromosomal DNA lacking a part or entire of the following (a) or (b):
(a) the gene encoding a protein having the amino acid sequence as shown in at least one of SEQ ID NOs: 1 and 2; or
(b) the gene encoding a protein having 80% or more homology with the amino acid sequence as shown in at least one of SEQ ID NOs: 1 and 2, and which has the ability to produce the amino acid,
provided that a protein having the amino acid sequence as shown in SEQ ID NO: 1 or 2 loses its activity due to such a lack.

2. The process according to claim 1, wherein the gene encoding a protein having the amino acid sequence as shown in SEQ ID NO: 1 or 2 has the nucleotide sequence as shown in SEQ ID NO: 3 or 4, respectively.

3. The process according to claim 1 or 2, wherein the ability to produce an amino acid is enhanced by a method selected from among the following [1] to [5]:
[1] a method for reducing or removing at least one mechanism for regulating biosynthesis of the amino acid;
[2] a method for increasing the expression level of at least one enzyme associated with biosynthesis of the amino acid;
[3] a method for increasing the copy number of at least one enzyme gene associated with biosynthesis of the amino acid;
[4] a method for attenuating or blocking at least one metabolic pathway which branches from the biosynthetic pathway of the amino acid into metabolites other than the amino acid; or
[5] a method for selecting a strain that exhibits higher resistance to an analogue of a amino acid than a wild-type strain.

4. The process according to any one of claims 1 to 3, wherein the microorganism is selected from the group consisting of *Erwinia berbicola, Erwinia amylovora, Erwinia carotovora, Serratia marcescens, Serratia ficaria, Serratia fonticola, Serratia liquefaciens, Salmonella typhimurium, Escherichia coli, Proteus rettgeri, Pseudomonas putida, Pseudomonas fluorescens, Pseudomonas aeruginosa, Pseudomonas dacunhae, Pseudomonas thazdinophilum, Xanthomonas oryzae*, and *Xanthomonas capestris.*

5. The process according to any one of claims 1 to 4, wherein the amino acid is proline or glutamic acid.

## Patentansprüche

1. Verfahren zum Herstellen einer Aminosäure, bei dem man einen Mirkoorganismus in einem Medium kultiviert, um so die Aminosäure in dem Kulturprodukt zu bilden und zu akkumulieren, und bei dem man die Aminosäure aus dem Kulturprodukt gewinnt, wobei der Mikroorganismus zu der Gattung *Erwinia, Serratia, Salmonella, Essherichia, Proteus, Pseudomonas oder Xanthomonas* gehört, und
wobei der Mikroorganismus chromosomale DNA umfasst, der das Folgende (a) oder (b) teilweise oder vollständig fehlt:
(a) das Gen, das ein Protein mit der Aminosäuresequenz kodiert, wie sie in wenigestens einer der SEQ ID Nr.: 1 und 2 dargestellt ist, oder
(b) das Gen, das ein Protein kodiert, das 80 % oder mehr Homologie mit der Aminosäuresequenz aufweist, wie sie in wenigstens einer der SEQ ID Nr.: 1 und 2 dargestellt ist, und welcher die Fähigkeit aufweist, die Aminosäure zu erzeugen,
Mit der Maßgabe, dass ein Protein mit der Aminosäuresequenz, wie sie In SEQ ID Nr.: 1 oder 2 dargestellt ist, seine Aktivität aufgrund eines solchen Mangels verziert.

2. Verfahren nach Anspruch 1, wobei das Gen, das ein Protein mit der Aminosäuresequenz, wie sie in SEQ ID Nr.: 1 oder 2 dargestellt ist, kodiert, jeweils die Nukleotidsequenz aufweist, wie sie in SEQ ID Nr.: 3 oder 4 dargestellt ist.

3. Verfahren nach Anspruch 1 oder 2, wobei die Fähigkeit, eine Aminosäure zu erzeugen, durch ein Verfahren verstärkt wird, das unter den folgenden [1] bis [5] ausgewählt ist
[1] ein Verfahren zum Verringern oder Entfernen wenigstens eines Mechanismus für die Regulierung der Biosynthese der Aminosäure,
[2] ein VerFahren zum Erhöhen des Expressionsnkaus von wenigstens einem mit der Biosynthese der Aminosäure assoziierte Enzyms,
[3] ein Verfahren zum Erhöhten der Kopienzahl von wenigstens einem Enzymgen, das mit der Biosynthese der Aminosäure assoziiert ist,
[4] ein Verfahren zum Attenuieren oder Blockieren wenigstens eines Stoffwechselweges, der von dem Biosyntheseweg der Aminosäure zu Metaboliten abzweigt, die anders als die Aminosäure sind, oder
[5] ein Verfahren zum Auswählen eines Stammes, der eine höhere Resistenz gegenüber einem Analogen einer Aminosäure aufweist als ein Witdtyp-Stamm.

4. Verfahren nach einem der Ansprüche 1 bis 3, wobei der Mikroorganismus ausgewählt ist aus der Gruppe, betstehend aus *Erwinia berbicola, Erwinia amylovora, Erwinia carotovora, Serratia marcescens, Serratia ficaria, Serratia fonticola, Serratia liquefaciens, Salmonella typhimurium, Escherichia coli, Proteus rettgeri, Pseudomonas putida, Pseudomonas fluorescens, Pseudomonas aeruginosa, Pseudomonas dacunhae, Pseudomonas thazdinophilum, Xanthomonas oryzae und Xanthomonas capestris.*

5. Verfahren nach einem der Ansprüche 1 bis 4, wobei die Aminosäure Prolin oder Glutaminsäure ist.

## Revendications

1. Procédé pour produire un acide aminé qui comprend la culture d'un microorganisme dans un milieu de sorte à former et à accumuler l'acide aminé dans le produit de culture et la récupération de l'acide aminé à partir du produit de culture, dans lequel le microorganisme appartient au genre *Erwinia, Serratia, Salmonella, Escherichia, Proteus, Pseudomonas* ou *Xanthomonas* et
dans lequel le microorganisme comprend de l'ADN chromosomique dans lequel une partie ou l'intégralité de (a) ou (b) ci-dessous est absente :
(a) le gène codant pour une protéine ayant la séquence d'acides aminés telle que montrée dans au moins une des SEQ ID NO: 1 et 2 ; ou
(b) le gène codant pour une protéine ayant 80 % d'homologie ou plus avec la séquence d'acides aminés telle que montrée dans au moins une des SEQ ID NO 1 et 2, et qui possède une aptitude à produire l'acide aminé,
à condition qu'une protéine ayant la séquence d'acides aminés telle que montrée dans SEQ ID NO: 1 ou 2 perde son activité en raison d'une telle absence.

2. Procédé selon la revendication 1, dans lequel le gène codant pour une protéine ayant la séquence d'acides aminés telle que montrée dans SEQ ID NO: 1 ou 2 possède la séquence nucléotidique telle que montrée dans SEQ ID NO: 3 ou 4, respectivement.

3. Procédé selon la revendication 1 ou 2, dans lequel l'aptitude à produire un acide aminé est améliorée par un procédé sélectionné parmi les procédés [1] à [5] suivants :
[1] un procédé pour réduire ou éliminer au moins un mécanisme régulant la biosynthèse de l'acide aminé ;
[2] un procédé pour augmenter le taux d'expression d'au moins une enzyme associée à la biosynthèse de l'acide aminé ;
[3] un procédé pour augmenter le nombre de copies d'au moins un gène enzymatique associé à la biosynthèse de l'acide aminé ;
[4] un procédé pour atténuer ou bloquer au moins une voie métabolique qui, à partir de la voie biosynthétique de l'acide aminé, se divise pour donner lieu à des métabolites autres que l'acide aminé ; ou
[5] un procédé pour sélectionner une souche qui exhibe une plus grande résistance à un analogue d'un acide aminé par rapport à une souche de type sauvage.

4. Procédé selon l'une quelconque des revendications 1 à 3, dans lequel le microorganism est sélectionné dans le groupe consistant en *Erwinia berbicola, Erwinia amylovora, Erwinia carotovora, Serratia marcescens, Serratia ficaria, Serratia fonticola, Serratia liquefaciens, Salmonella typhimurium, Escherichia coli, Proteus rettgeri, Pseudomonas putida, Pseudomonas fluorescens, Pseudomonas aeruginosa, Pseudomonas dacunhae, Pseudomonas thazdinophilum, Xanthomonas oryzae*, et *Xanthomonas capestris.*

5. Procédé selon l'une quelconque des revendications 1 à 4, dans lequel l'acide aminé est la proline ou l'acide glutamique.


### SEQUENCE LISTING

<110> KYOWA HAKKO KOGYO CO., LTD.
<120> Industrially Useful Microorganism
<130> P028609EP
<140> 05776635.4
   <141> 2005-09-01
<150> JP 254446/2004
   <151> 2004-09-01
<160> 38
<170> Patent In Version 2.1
<210> 1
   <211> 99
   <212> PRT
   <213> Escherichia coli
<400> 1
   Met Ala Leu Thr Lys Ala Glu Met Ser Glu Tyr Leu Phe Asp Lys Leu
    1               5                  10                  15
   Gly Leu Ser Lys Arg Asp Ala Lys Glu Leu Val Glu Leu Phe Phe Glu
               20                  25                  30
   Glu Ile Arg Arg Ala Leu Glu Asn Gly Glu Gln Val Lys Leu Ser Gly
           35                  40                  45
   Phe Gly Asn Phe Asp Leu Arg Asp Lys Asn Gln Arg Pro Gly Arg Asn
       50                  55                  60
   Pro Lys Thr Gly Glu Asp Ile Pro Ile Thr Ala Arg Arg Val Val Thr
   65                  70                  75                  80
   Phe Arg Pro Gly Gln Lys Leu Lys Ser Arg Val Glu Asn Ala Ser Pro
                   85                  90                  95
   Lys Asp Glu
<210> 2
   <211> 94
   <212> PRT
   <213> Escherichia coli
<400> 2
   Met Thr Lys Ser Glu Leu Ile Glu Arg Leu Ala Thr Gln Gln Ser His
    1               5                  10                  15
   Ile Pro Ala Lys Thr Val Glu Asp Ala Val Lys Glu Met Leu Glu His
               20                  25                  30
   Met Ala Ser Thr Leu Ala Gln Gly Glu Arg Ile Glu Ile Arg Gly Phe
           35                  40                  45
   Gly Ser Phe Ser Leu His Tyr Arg Ala Pro Arg Thr Gly Arg Asn Pro
       50                  55                  60
   Lys Thr Gly Asp Lys Val Glu Leu Glu Gly Lys Tyr Val Pro His Phe
   65                  70                  75                  80
   Lys Pro Gly Lys Glu Leu Arg Asp Arg Ala Asn Ile Tyr Gly
                   85                  90
<210> 3
   <211> 597
   <212> DNA
   <213> Escherichia coli
<400> 3
   gcgagatttc tcgcttcccg gcgaaccgtc gtgacatcgc ggtggtggtc gcagaaaacg 60
   ttcccgcagc ggatatttta tccgaatgta agaaagttgg cgtaaatcag gtagttggcg 120
   taaacttatt tgacgtgtac cgcggtaagg gtgttgcgga ggggtataag agcctcgcca 180
   taagcctgat cctgcaagat accagccgta cactcgaaga agaggagatt gccgctaccg 240
   tcgccaaatg tgtagaggca ttaaaagagc gattccaggc atcattgagg gattgaacct 300
   atg gcg ctt aca aaa gct gaa atg tca gaa tat ctg ttt gat aag ctt   348
   Met Ala Leu Thr Lys Ala Glu Met Ser Glu Tyr Leu Phe Asp Lys Leu
    1               5                  10                  15
   ggg ctt agc aag cgg gat gcc aaa gaa ctg gtt gaa ctg ttt ttc gaa   396
   Gly Leu Ser Lys Arg Asp Ala Lys Glu Leu Val Glu Leu Phe Phe Glu
               20                  25                  30
   gag atc cgt cgc gct ctg gaa aac ggc gaa cag gtg aaa ctc tct ggt   444
   Glu Ile Arg Arg Ala Leu Glu Asn Gly Glu Gln Val Lys Leu Ser Gly
           35                  40                  45
   ttt ggt aac ttc gat ctg cgt gat aag aat caa cgc ccg gga cgt aac   492
   Phe Gly Asn Phe Asp Leu Arg Asp Lys Asn Gln Arg Pro Gly Arg Asn
       50                  55                  60
   ccg aaa acg ggc gag gat att ccc att aca gca cgg cgc gtg gtg acc   540
   Pro Lys Thr Gly Glu Asp Ile Pro Ile Thr Ala Arg Arg Val Val Thr
   65                  70                  75                  80
   ttc aga ccc ggg cag aag tta aaa agc cgg gtc gaa aac gct tcg ccc   588
   Phe Arg Pro Gly Gln Lys Leu Lys Ser Arg Val Glu Asn Ala Ser Pro
                   85                  90                  95
   aaa gac gag                                                       597
   Lys Asp Glu
<210> 4
   <211> 582
   <212> DNA
   <213> Escherichia coli
<400> 4
   aaccgcgcaa tcagcctgtc tgttcgtgcg aaagacgaag ctgacgagaa agatgcaatc 60
   gcaactgtta acaaacagga agatgcaaac ttctccaaca acgcaatggc tgaagctttc 120
   aaagcagcta aaggcgagta attctctgac tcttcgggat ttttattccg aagtttgttg 180
   agtttacttg acagattgca ggtttcgtcc tgtaatcaag cactaagggc ggctacggcc 240
   gcccttaatc aatgcagcaa cagcagccgc ttaatttgcc tttaaggaac cggaggaatc 300
   atg acc aag tca gaa ttg ata gaa aga ctt gcc acc cag caa tcg cac   348
   Met Thr Lys Ser Glu Leu Ile Glu Arg Leu Ala Thr Gln Gln Ser His
   1               5                   10                  15
   att ccc gcc aag acg gtt gaa gat gca gta aaa gag atg ctg gag cat   396
   Ile Pro Ala Lys Thr Val Glu Asp Ala Val Lys Glu Met Leu Glu His
               20                  25                  30
   atg gcc tcg act ctt gcg cag ggc gag cgt att gaa atc cgc ggt ttc   444
   Met Ala Ser Thr Leu Ala Gln Gly Glu Arg Ile Glu Ile Arg Gly Phe
           35                  40                  45
   ggc agt ttc tct ttg cac tac cgc gca cca cgt acc gga cgt aat ccg   492
   Gly Ser Phe Ser Leu His Tyr Arg Ala Pro Arg Thr Gly Arg Asn Pro
       50                  55                  60
   aag act ggc gat aaa gta gaa ctg gaa gga aaa tac gtt cct cac ttt   540
   Lys Thr Gly Asp Lys Val Glu Leu Glu Gly Lys Tyr Val Pro His Phe
   65                  70                  75                  80
   aaa cct ggt aaa gaa ctg cgc gat cgc gcc aat att tac ggt           582
   Lys Pro Gly Lys Glu Leu Arg Asp Arg Ala Asn Ile Tyr Gly
                   85                  90
<210> 5
   <211> 34
   <212> DNA
   <213> Artificial
<220>
   <223> Synthetic DNA
<400> 5
   gaagttccta tactttctag agaataggaa cttc 34
<210> 6
   <211> 25
   <212> DNA
   <213> Artificial
<220>
   <223> Synthetic DNA
<400> 6
   gtctccgaac ttttccctaa cgaag 25
<210> 7
   <211> 49
   <212> DNA
   <213> Artificial
<220>
   <223> Synthetic DNA
<400> 7
   acccagcctg cgcgagcagg ggaataataa acgtaattgc cggatgcga 49
<210> 8
   <211> 48
   <212> DNA
   <213> Artificial
<220>
   <223> Synthetic DNA
<400> 8
   attcccctgc tcgcgcaggc tgggtaggcc aatttattgc tatttacc 48
<210> 9
   <211> 47
   <212> DNA
   <213> Artificial
<220>
   <223> Synthetic DNA
<400> 9
   ttatttgccg actaccttgg tgatcagctc taatgcgctg ttaatca 47
<210> 10
   <211> 50
   <212> DNA
   <213> Artificial
<220>
   <223> Synthetic DNA
<400> 10
   gatcaccaag gtagtcggca aataaacgtt tcccgttgaa tatggctcat 50
<210> 11
   <211> 24
   <212> DNA
   <213> Artificial
<220>
   <223> Synthetic DNA
<400> 11
   tttgtttgcg tttactggca gata 24
<210> 12
   <211> 25
   <212> DNA
   <213> Artificial
<220>
   <223> Synthetic DNA
<400> 12
   gtctccgaac ttttccctaa cgaag 25
<210> 13
   <211> 25
   <212> DNA
   <213> Artificial
<220>
   <223> Synthetic DNA
<400> 13
   ttagaaaaac tcatcgagca tcaaa 25
<210> 14
   <211> 24
   <212> DNA
   <213> Artificial
<220>
   <223> Synthetic DNA
<400> 14
   acggaagatc acttcgcaga ataa 24
<210> 15
   <211> 24
   <212> DNA
   <213> Artificial
<220>
   <223> Synthetic DNA
<400> 15
   attaattgcg ttgcgctcac tgcc 24
<210> 16
   <211> 74
   <212> DNA
   <213> Artificial
<220>
   <223> Synthetic DNA
<400> 16
   atgggaacca ccaccatggg ggttaagctg gacgacgcga atgagacgtt gatcggcacg 60
   taagaggttc caac 74
<210> 17
   <211> 56
   <212> DNA
   <213> Artificial
<220>
   <223> Synthetic DNA
<400> 17
   ttaacctata gtcattaagc tggcgttacc gccagcggca tttctgccat tcatcc 56
<210> 18
   <211> 40
   <212> DNA
   <213> Artificial
<220>
   <223> Synthetic DNA
<400> 18
   atgggaacca ccaccatggg ggttaagctg gacgacgcga 40
<210> 19
   <211> 40
   <212> DNA
   <213> Artificial
<220>
   <223> Synthetic DNA
<400> 19
   tgccgctggc ggtaacgcca gcttaatgac tataggttaa 40
<210> 20
   <211> 47
   <212> DNA
   <213> Artificial
<220>
   <223> Synthetic DNA
<400> 20
   attcccctgc tcgcgcaggc tgggtcgcca gccaggacag aaatgcc 47
<210> 21
   <211> 47
   <212> DNA
   <213> Artificial
<220>
   <223> Synthetic DNA
<400> 21
   ttatttgccg actaccttgg tgatcgtcgt attaaagagg ggcgtgg 47
<210> 22
   <211> 65
   <212> DNA
   <213> Artificial
<220>
   <223> Synthetic DNA
<400> 22
   gtttgatatc atttttccta aaattgaatg gcagagaatc attcccctgc tcgcgcaggc 60
   tgggt 65
<210> 23
   <211> 65
   <212> DNA
   <213> Artificial
<220>
   <223> Synthetic DNA
<400> 23
   ttgtgaatca aatggctact tttgcatcac ccggttttat ttatttgccg actaccttgg 60
   tgatc 65
<210> 24
   <211> 40
   <212> DNA
   <213> Artificial
<220>
   <223> Synthetic DNA
<400> 24
   gtttgatatc atttttccta aaattgaatg gcagagaatc 40
<210> 25
   <211> 40
   <212> DNA
   <213> Artificial
<220>
   <223> Synthetic DNA
<400> 25
   ataaaaccgg gtgatgcaaa agtagccatt tgattcacaa 40
<210> 26
   <211> 25
   <212> DNA
   <213> Artificial
<220>
   <223> Synthetic DNA
<400> 26
   tcgtatttca gacctgttgc ccatg 25
<210> 27
   <211> 24
   <212> DNA
   <213> Artificial
<220>
   <223> Synthetic DNA
<400> 27
   gattctctgc cattcaattt tagg 24
<210> 28
   <211> 24
   <212> DNA
   <213> Artificial
<220>
   <223> Synthetic DNA
<400> 28
   aaaccgggtg atgcaaaagt agcc 24
<210> 29
   <211> 23
   <212> DNA
   <213> Artificial
<220>
   <223> Synthetic DNA
<400> 29
   tgcgaagccg acacccttgg cag 23
<210> 30
   <211> 40
   <212> DNA
   <213> Artificial
<220>
   <223> Synthetic DNA
<400> 30
   aaatgctgct gacccgtgct aatatggaag accgtgaacg 40
<210> 31
   <211> 50
   <212> DNA
   <213> Artificial
<220>
   <223> Synthetic DNA
<400> 31
   ttatttgccg actaccttgg tgatctgtgt ctcaaaatct ctgatgttac 50
<210> 32
   <211> 50
   <212> DNA
   <213> Artificial
<220>
   <223> Synthetic DNA
<400> 32
   attcccctgc tcgcgcaggc tgggtttaga aaaactcatc gagcatcaaa 50
<210> 33
   <211> 25
   <212> DNA
   <213> Artificial
<220>
   <223> Synthetic DNA
<400> 33
   ttatttgccg actaccttgg tgatc 25
<210> 34
   <211> 25
   <212> DNA
   <213> Artificial
<220>
   <223> Synthetic DNA
<400> 34
   acccagcctg cgggagcagg ggaat 25
<210> 35
   <211> 65
   <212> DNA
   <213> Artificial
<220>
   <223> Synthetic DNA
<400> 35
   ttaaaagagc gattccaggc atcattgagg gattgaacct ttatttgccg actaccttgg 60
   tgatc 65
<210> 36
   <211> 65
   <212> DNA
   <213> Artificial
<220>
   <223> Synthetic DNA
<400> 36
   ttactcgtct ttgggcgaag cgttttcgac ccggcttttt attcccctgc tcgcgcaggc 60
   tgggt 65
<210> 37
   <211> 65
   <212> DNA
   <213> Artificial
<220>
   <223> Synthetic DNA
<400> 37
   cagcagccgc ttaatttgcc tttaaggaac cggaggaatc ttatttgccg actaccttgg 60
   tgatc 65
<210> 38
   <211> 65
   <212> DNA
   <213> Artificial
<220>
   <223> Synthetic DNA
<400> 38
   cgacaggtgc ttttctctcg ttcaagtttg agtaaaaaac attcccctgc tcgcgcaggc 60
   tgggt 65